# EUROPÄISCHE PATENTSCHRIFT

(11) **EP 0 833 616 B1**
(45) Veröffentlichungstag und Bekanntmachung des Hinweises auf die Patenterteilung: **04.10.2001**
(21) Anmeldenummer: 96917391.3
(22) Anmeldetag: 21.05.1996
(51) Int. Cl.: A61K 9/16, A61K 9/20

(54) **VERFAHREN ZUM KONTINUIERLICHEN ERSINTERN EINES GRANULATS**
PROCESS FOR THE CONTINUOUS AGGLOMERATION OF GRANULES
PROCEDE D'AGGLOMERATION CONTINU DE GRANULES

(30) Priorität: 23.06.1995 DE 19522899
(43) Veröffentlichungstag der Anmeldung: 08.04.1998
(73) Patentinhaber: HEXAL AG, D-83607 Holzkirchen (DE)
(72) Erfinder: KNIDLBERGER, Astrid, D-83607 Holzkirchen (DE)
(74) Vertreter: Boeters, Hans Dietrich, Dr.
(86) Internationale Anmeldenummer: EP9602177
(87) Internationale Veröffentlichungsnummer: WO9700673

(56) Entgegenhaltungen:
- EP-A- 0 043 254
- WO-A-93/24110
- WO-A-95/09044

## Beschreibung

EP-B-0 043 254 beschreibt ein Verfahren zur Herstellung von Arzneimittelzubereitungen mit verzögerter Wirkstoffabgabe, das auf einem selektiven Aufschmelzprozeß mindestens zweier lipider oder lipoider Komponenten beruht, die eine retardierende Wirkung für mit diesen Komponenten vermischte Arzneimittelwirkstoffe haben. Bei diesem bekannten Verfahren wird
(a) der Wirkstoff fein verteilt,
(b) der fein verteilte Wirkstoff sowohl mit einer fein verteilten hochschmelzenden lipiden oder lipoiden Komponente als auch mit einer fein verteilten niedrigschmelzenden lipiden oder lipoiden Komponente vermischt, wobei das Gewichtsverhältnis der beiden lipiden oder lipoiden Komponenten im Bereich von 1 : 5 bis 5 : 1 liegt,
(c) das resultierende Gemisch aus Wirkstoff und lipiden oder lipoiden Komponenten auf eine Temperatur gebracht, die oberhalb des Schmelzpunkts der niedrigschmelzenden Komponente, jedoch unterhalb des Schmelzpunkts der hochschmelzenden Komponente liegt, wobei der Wirkstoff und die hochschmelzende lipide oder lipoide Komponente gleichmäßig in der vollständig geschmolzenen niedrigschmelzenden lipiden oder lipoiden Komponente dispergiert werden,
(d) das resultierende Gemisch nach dem Aufschmelzen der niedrigschmelzenden Komponente unter deren Schmelzpunkt abgekühlt und
(e) das resultierende Gemisch während des Abkühlens oder danach granuliert.

WO-A-93/24110 beschreibt ein Verfahren zur Herstellung retardierter Arzneimittelzubereitungen, bei dem man die einzelnen Komponenten
(i) mit Hilfe eines Extruders als teilaufgeschmolzenes Produkt durch eine Düsenplatte extrudiert,
(ii) das Extrudat in Form von Strängen abkühlt,
(iii) einem Granulator zuführt und
(iv) das fertige Granulat einer Tablettierung unterwirft.

Wenn man jedoch nach der EP-B-0 043 254 die niedrigschmelzende lipide oder lipoide Komponente vollständig schmilzt, so können sich Modifikationen dieser Komponenten bzw. Fettmodifikationen bilden, die sich später beim Lagern von Tabletten, die unter Verwendung des bekannten Granulats hergestellt worden sind, derart rückwandeln können, daß die Wirkstoffreisetzung beeinflußt wird. Bei dem aus WO-A-93/24110 bekannten Verfahren kann es wiederum zu Schereffekten kommen, die zum Materialabrieb im Extruderrohr oder an der Düsenplatte und damit zu einem unerwünschten Metalleintrag in das Extrudat führen können.

Aufgabe der vorliegenden Erfindung ist es, den geschilderten Stand der Technik zu verbessern.

Dazu wird erfindungsgemäß ein Verfahren zum kontinuierlichen Ersintern eines Granulats für die Herstellung von Preßlingen, insbesondere von Arzneimittelzubereitungen in Form von Tabletten, vorgesehen, bei dem man
(a) die einzelnen Komponenten zu einem Pulver mischt,
(b) das anfallende Pulver in trockenem Zustand in einen Extruder gibt,
(c) das aufgegebene Pulver mit Hilfe einer Förderschnecke in Richtung auf die offene Extruderstirn bzw. den Auswurf fördert,
(d) wobei man den Extruder und/oder die Schnecke erwärmt und eine oder einige der Komponenten anteigt oder erweicht (jedenfalls keine der Komponenten schmilzt) und Partikel des Pulvergemischs zu einem Granulat verkleben oder versintern läßt,
(e) das anfallende ersinterte Granulat gegebenenfalls siebt und
(f) in an sich bekannter Weise tablettiert.

Das beanspruchte Verfahren arbeitet also drucklos, da eine Düse fehlt. Dadurch wird das Auftreten von Scherkräften, wie beim bekannten Extrudieren, weitgehend vermieden, so daß es praktisch zu keinem Abrieb des Extruderrohres oder an einer beim Stand der Technik vorgesehenen Düse und damit zu keinem Metalleintrag kommt. Da nicht eine einzige der eingesetzten Komponenten geschmolzen wird und da auch kein Strang extrudiert wird, fällt das aus dem Extruder austretende Material beim erfindungsgemäßen Verfahren als Granulat an, so daß es nicht einer gesonderten Granulierstufe unterworfen werden muß. Stattdessen kann das aus dem Extruder austretende Material erforderlichenfalls gesiebt werden, wobei der Siebrückstand der Tablettierung zugeführt wird, während das durchgesiebte Material erneut in das erfindungsgemäße Verfahren eingespeist werden kann.

Als eine der Komponenten für das Pulver, das in den Extruder gegeben wird, kann man eine lipide oder lipoide Komponente verwenden.

Bei erfindungsgemäßen Verfahren kann man von Komponenten ausgehen, die einen Wirkstoff umfassen, der eine Wasserlöslichkeit > 0,5 % aufweist. Beispiele für Wirkstoffe sind Diltiazem, Sotalol, Diclofenac oder eines ihrer Derivate, wie Diltiazem.HCl, Sotalol.HCl oder Diclofenac-Natrium.

Beim erfindungsgemäßen Verfahren kann man den Extruder und/oder die Schnecke zonenweise erwärmen, wobei (in Förderrichtung) drei oder mehr Zonen aufeinanderfolgen, von denen die mittlere Zone (n) eine höhere Temperatur als die flankierenden Zonen aufweist (aufweisen).

Erfindungsgemäß kann man eine Schnecke verwenden, bei der (in Förderrichtung) auf eine Zone mit vorgegebener Windungsbreite und Windungssteigung eine Zone mit größerer Windungsbreite und/oder flacherer Windungssteigung folgt. Dabei lassen sich die Zonen unterschiedlicher Windungsbreite und/oder Windungssteigung beispielsweise mit Hilfe von miteinander verbundenen Einzelelementen der Schnecke vorsehen.

Erfindungsgemäß kann man als Schnecke auch zwei zueinander parallel angeordnete kämmende Schneckenkörper im Gleichlauf oder Gegenlauf vorsehen.

Nachstehend wird die Erfindung durch Beispiele näher erläutert.

### Beispiel 1 (Diltiazem 90 mg Retardtablette)

Die Komponenten der inneren Phase wurden erforderlichenfalls gesiebt, eingewogen und gemischt. Für die Komponenten der Außenphase verfuhr man analog. Dabei wurden folgende Rezepturen gewählt.

| | | |
|---|---|---|
| Innere Phase | Diltiazem.HCl | 90,00 |
| | Cutina HR | 100,00 |
| | Lactose | 30,00 |
| | Polyvidon | 20,00 |
| | PEG | 20,00 |
| Außenphase | mikrokristalline Cellulose | 62,00 |
| | Na-carboxymethylstärke | 1,70 |
| | hochdisperses Siliciumdioxid | 3,30 |
| | Magnesiumstearat | 3,00 |
| Summe: | | 330,00 |

Die Mischung der Innenphase wurde in den Vorratsbehälter eines Extruders eingebracht. Der Extruder wurde ohne Lochscheibe betrieben, so daß sich im Extrusionszylinder kein Druck aufbaute. Bei dem verwendeten Extruder folgten in Förderrichtung vier Temperaturzonen folgendermaßen aufeinander:

| Zone 1 | Zone 2 | Zone 3 | Zone 4 |
|---|---|---|---|
| 42 ± 5°C | 68 ± 5°C | 72 ± 5°C | 57 ± 5°C |

Die einzelnen Zonen wurden auf die angegebenen Temperaturen aufgeheizt, wobei man die Mischung für die innere Phase durch den Extruder gab, nachdem die angegebenen Temperaturen erreicht worden waren. Am Austrag fiel ein Granulat an, das sich sieben ließ, und das mit der Mischung für die Außenphase gemischt wurde. Diese Mischung wurde zu Tabletten verpreßt.

### Beispiel 2 (Diltiazem 120 mg Retardtablette)

Man ging wie in Beispiel 1 vor, wobei man jedoch für die innere Phase und die Außenphase folgende Rezepturen und für die vier aufeinanderfolgenden Temperaturzonen folgende Temperaturen vorsah.

| | | |
|---|---|---|
| Innere Phase | Diltiazem.HCl | 120,00 |
| | Cutina HR | 46,00 |
| | Lactose | 208,00 |
| | Stearinsäure | 70,00 |
| Außenphase | Hydroxyethylcellulose | 3,20 |
| | Magnesiumstearat | 1,50 |
| Summe: | | 448,70 |

| | | | |
|---|---|---|---|
| Zone 1 | Zone 2 | Zone 3 | Zone 4 |
| 44 ± 5°C | 69 ± 5°C | 70 ± 5°C | 52 ± 5°C |

### Beispiel 3 (Sotalol 240 mg Retardtablette)

Man folgte Beispiel 1, wobei allerdings eine 2-Schichttablette mit folgenden Rezepturen hergestellt und folgende Temperaturen für die aufeinanderfolgenden Temperaturzonen vorgesehen wurden.

| | | |
|---|---|---|
| Initialschicht | Sotalol.HCl | 40,0 |
| | Lactose | 30,0 |
| | Maisstärke | 30,0 |
| | Hydroxypropylcellulose | 3,0 |
| | Blaulack Gereinigtes Wasser q.s. | 0,12 |
| | Na-carboxymethylstärke | 15,0 |
| | Lactose | 35,0 |
| | hochdisperses Siliciumdioxid | 1,0 |
| | Magnesiumstearat | 2,0 |
| Summe: | | 156,12 |
| Retardschicht Innere Phase | | |
| | Sotalol.HCl | 200,0 |
| | Lactose | 100,0 |
| | Cutina HR | 140,0 |
| Außenphase | Magnesiumstearat | 4,0 |
| Summe | | 444,0 |

| | | | |
|---|---|---|---|
| Zone 1 | Zone 2 | Zone 3 | Zone 4 |
| 50 ± 5°C | 71 ± 5°C | 69 ± 5°C | 49 ± 5°C |

### Beispiel 4 (Diclofenac 100 mg Retardtablette)

Man folgte Beispiel 1, wobei man jedoch für die innere Phase und die Außenphase folgende Rezepturen und für die vier aufeinanderfolgenden Temperaturzonen folgende Temperaturen vorsah.

| | | |
|---|---|---|
| Innere Phase | Diclofenac-Natrium | 100,00 |
| | Saccharose | 105,00 |
| | 1-Hexadecanol | 55,20 |
| Außenphase | hochdisperses Siliciumdioxid | 0,52 |
| | Magnesiumstearat | 1,30 |
| | Poly-(1-vinyl-2-pyrrolidon) | 1,28 |

| Zone 1 | Zone 2 | Zone 3 | Zone 4 |
|---|---|---|---|
| 50 ± 5°C | 64 ± 5°C | 63 ± 5°C | 48 ± 5°C |

## Patentansprüche

1. Verfahren zum kontinuierlichen Ersintern eines Granulats für die Herstellung von Preßlingen, bei dem man
(a) die einzelnen Komponenten zu einem Pulver mischt,
(b) das anfallende Pulver in trockenem Zustand in einen Extruder gibt,
(c) das aufgegebene Pulver mit Hilfe einer Förderschnecke in Richtung auf die offene Extruderstirn fördert,
(d) wobei man den Extruder und/oder die Schnecke erwärmt und eine oder einige der Komponenten des Pulvergemischs erweicht oder schmilzt und Partikel des Pulvergemischs zu einem Granulat verklebt,
(e) das anfallende Granulat gegebenenfalls siebt und
(f) in an sich bekannter Weise tablettiert.

2. Verfahren nach Anspruch 1, **dadurch gekennzeichnet, daß** man als eine der Komponenten für das Pulver eine lipide oder lipoide Komponente verwendet.

3. Verfahren nach Anspruch 1 oder 2, **dadurch gekennzeichnet, daß** man von Komponenten ausgeht, die einen Wirkstoff umfassen, der eine Wasserlöslichkeit > 0,5 % aufweist.

4. Verfahren nach einem der vorhergehenden Ansprüche, **dadurch gekennzeichnet, daß** man als Wirkstoff Diltiazem, Sotalol, Diclofenac oder ein Derivat dieser Wirkstoffe einsetzt.

5. Verfahren nach einem der vorhergehenden Ansprüche, **dadurch gekennzeichnet, daß** man den Extruder und/oder die Schnecke zonenweise erwärmt, wobei (in Förderrichtung) drei oder mehr Zonen aufeinander folgen, von denen die mittlere Zone (n) eine höhere Temperatur als die flankierenden Zonen aufweist (aufweisen).

6. Verfahren nach einem der vorhergehenden Ansprüche, **dadurch gekennzeichnet, daß** man eine Schnecke verwendet, bei der (in Förderrichtung) auf eine Zone mit vorgegebener Windungsbreite und Windungssteigung eine Zone mit größerer Windungsbreite und/oder flacherer Windungssteigung folgt.

7. Verfahren nach einem der vorhergehenden Ansprüche, **dadurch gekennzeichnet, daß** man die Zonen unterschiedlicher Windungsbreite und/oder Windungssteigung mit Hilfe von miteinander verbundenen Einzelelementen der Schnecke vorsieht.

8. Verfahren nach einem der vorhergehenden Ansprüche, **dadurch gekennzeichnet, daß** man als Schnecke zwei parallel zueinander angeordnete kämmende Schneckenkörper im Gleichlauf ohne Gegenlauf vorsieht.

## Claims

1. Process for continuous sintering of granules for the preparation of pellets, in which
(a) the individual components are mixed to form a powder,
(b) the powder obtained is introduced in the dry state into an extruder,
(c) the powder introduced is conveyed with the aid of a conveying screw in the direction of the open extruder front,
(d) the extruder and/or the screw being heated and one or some of the components of the powder mixture softening or meting and particles of the powder mixture sticking together to form granules,
(e) if appropriate, the granules obtained are sieved and
(f) tabletting is carried out in a manner known per se.

2. Process according to claim 1, **characterized in that** a lipid or lipoid component is used as one of the components for the powder.

3. Process according to claim 1 or 2, **characterized in that** components which comprise an active compound which has a water-solubility of > 0.5 % are used as starting substances.

4. Process according to one of the preceding claims, **characterized in that** Diltiazem, Sotalol, Diclofenac or a derivative of the active compounds is employed as the active compound.

5. Process according to one of the preceding claims, **characterized in that** the extruder and/or the screw is heated in zones, three or more zones following one another (in the conveying direction), the middle zone(s) of which has (have) a higher temperature than the flanking zones.

6. Process according to one of the preceding claims, **characterized in that** a screw is used in which (in the conveying direction) a zone of given thread width and thread pitch is followed by a zone of higher thread width and/or flatter thread pitch.

7. Process according to one of the preceding claims, **characterized in that** the zones of different thread width and/or thread pitch are provided with the aid of individual elements of the screw connected to one another.

8. Process according to one of the preceding claims, **characterized in that** two combing screw bodies arranged parallel to one another and running in the same direction without running in the opposite direction are provided as the screw.

## Revendications

1. Procédé de frittage en continu d'un granulat pour la fabrication de comprimés dans lequel on :
(a) mélange les divers constituants pour en faire une poudre,
(b) charge la poudre produite à l'état sec dans une extrudeuse,
(c) amène la poudre chargée à l'aide d'une vis sans fin en direction du front ouvert de l'extrudeuse,
(d) tout en réchauffant l'extrudeuse et/ou la vis sans fin et en ramollissant ou en faisant fondre un ou quelques-uns des constituants du mélange de poudre et en collant des particules du mélange de poudre pour en faire un granulat,
(e) filtre aussi le granulat produit et
(f) produit des comprimés d'une manière connue en elle-même.

2. Procédé selon la revendication 1, **caractérisé en ce que** l'on utilise un constituant lipide ou lipoïde comme un des constituants pour la poudre.

3. Procédé selon la revendication 1 ou 2, **caractérisé en ce que** l'on part de constituants qui comprennent une matière active présentant une solubilité dans l'eau > 0,5 %.

4. Procédé selon l'une quelconque des revendications précédentes, **caractérisé en ce que** l'on utilise comme matière active Diltiazem, Sotalol, Diclofenac ou un dérivé de ces matières actives.

5. Procédé selon l'une quelconque des revendications précédentes, **caractérisé en ce que** l'extrudeuse et/ou la vis sans fin sont réchauffées zone par zone, trois zones ou plus étant disposées l'une après l'autre (dans le sens d'avancement) et la (les) zone(s) centrale(s) présentant une température plus élevée que les zones adjacentes.

6. Procédé selon l'une quelconque des revendications précédentes, **caractérisé en ce que** l'on utilise une vis sans fin dans laquelle (dans le sens d'avancement) une zone avec une largeur de spire et un pas de spire prédéfinis est suivie d'une zone avec une largeur de spire plus grande et/ou un pas de spire plus plat.

7. Procédé selon l'une quelconque des revendications précédentes, **caractérisé en ce que** l'on prévoit les zones ayant une largeur de spire et/ou un pas de spire différents à l'aide d'éléments individuels de la vis sans fin connectés les uns aux autres,

8. Procédé selon l'une quelconque des revendications précédentes, **caractérisé en ce que** l'on prévoit comme vis sans fin deux corps de vis sans fin peigneurs disposés parallèlement l'un à l'autre et synchronisés sans rotation antagoniste.
